Europäisches Patentamt

(19) European Patent Office

Office européen des brevets

(11) Publication number:

**0 078 035**
**B1**

## (12) EUROPEAN PATENT SPECIFICATION

(45) Date of publication of patent specification: **17.09.86**

(21) Application number: **82109786.2**

(22) Date of filing: **22.10.82**

(51) Int. Cl.⁴: **A 61 B 1/00,** A 61 B 1/06, A 61 B 1/12

(54) Endoscope system.

(30) Priority: **22.10.81 JP 168947/81**
**05.11.81 JP 177751/81**

(43) Date of publication of application:
**04.05.83 Bulletin 83/18**

(45) Publication of the grant of the patent:
**17.09.86 Bulletin 86/38**

(84) Designated Contracting States:
**AT BE CH DE FR GB IT LI NL SE**

(56) References cited:
**EP-A-0 026 497**
**DE-A-2 949 827**

(73) Proprietor: **OLYMPUS OPTICAL CO., LTD.**
**43-2, 2-chome, Hatagaya Shibuya-ku**
**Tokyo 151 (JP)**

(72) Inventor: **Tsukaya, Takashi**
**4-22-13, Oowada-machi**
**Hachioji-shi Tokyo (JP)**
Inventor: **Takamatsu, Tokuyuki**
**1-17-8, Higashikoiwa**
**Edogawa-ku Tokyo (JP)**

(74) Representative: **Kuhnen, Wacker & Partner**
**Schneggstrasse 3-5 Postfach 1729**
**D-8050 Freising (DE)**

Courier Press, Leamington Spa, England.

## Description

This invention relates to a safety circuit for an endoscope system, particularly for a safety circuit for a microprocessor controlled system.

Recently, microcomputers have come to be applied to uses in a wide variety of apparatus and systems including endoscope systems. Application of a microcomputer to the use in an endoscope system requires at least the minimum of a safe measure to counter run over of a CPU of the microcomputer due to noise or heat, since the endoscope of the system is used for diagnosis on the human body or the like. Especially in those endoscopes which include air and water supply units or a light source unit, if the CPU runs over, air and water supply will be rendered unstable, or illumination light will fail to be supplied to the affected region to be examined. Thus, the affected region cannot be observed through the eyepiece section of the endoscope and this would be a serious situation from a viewpoint of security.

In an endoscope with a light source unit, moreover, run over of the CPU will make unstable a lamp current to be supplied to a source lamp, or cause a color temperature changing filter or shutter to intercept a source light path, thereby preventing a region of interest from being supplied with sufficient illumination light. Thus, it is impossible distinctly to perceive the region of interest through the eyepiece section of the endoscope and this would also lead to a security risk.

Various attempts have been made in the past time to overcome the above-mentioned problems. For example from EP—AI 0026497 an endoscope system is known, wherein an endoscope, a light source unit and a photographic attachment are connected to a centralized supervision-control device which collectively supervises and controls the overall operating condition of the endoscope, the light source unit and the photographic attachment. However, there are no provisions against failure of the supervision-control device itself.

The basic idea to provide a digital control unit, especially a microcomputer control unit comprising a microprocessor with a failure detection arrangement is known for example from DE—A—2 949 827.

This known arrangement, however, works only in combination with a corresponding specific software and therefore is not applicable to the wide technical field of endoscopic systems.

It is therefore an object of the invention to provide a safety circuit for a microprocessor controlled endoscope system with a CPU ensuring at least minimum of safety even in case of wrong operation of the CPU.

The solution of this object is achieved by the characterizing features of claim 1 and 5, respectively.

The related subclaims contain preferred embodiments of the invention.

This invention can be more fully understood from the following detailed description when taken in conjunction with the accompanying drawings, in which:

Fig. 1 is a block diagram showing an endoscope system with a CPU according to one embodiment of this invention;

Figs. 2A to 2H show a timing chart for illustrating the operation of the endoscope shown in Fig. 1;

Figs. 3A to 3G show a timing chart of a microcomputer in the endoscope system of Fig. 1 obtained when the microcomputer operates normally;

Figs. 4A to 4G show a timing chart of the microcomputer obtained when the microcomputer runs over;

Fig. 5 is a block diagram showing an endoscope system with a CPU according to another embodiment of the invention; and

Fig. 6 is a block diagram of the system shown in Fig. 5.

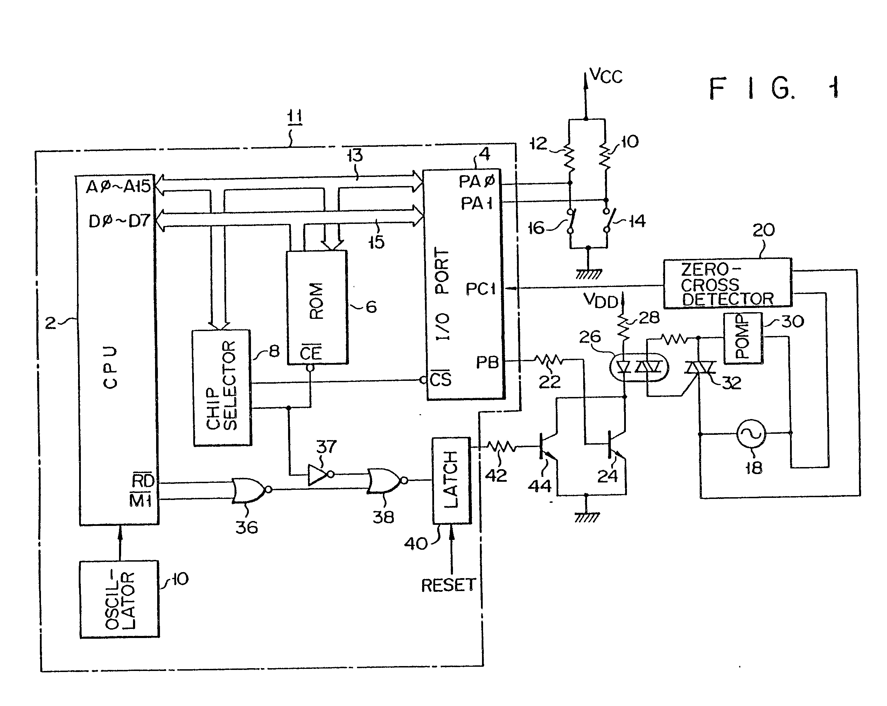

As shown in Fig. 1, a microcomputer 11 is disposed in a light source unit (not shown). The microcomputer 11 comprises a CPU 2, an I/O port 4, an ROM 6, a chip selector 8, and an oscillator 10. Address terminals A$\phi$ to A15 of the CPU 2 are connected to the I/O port 4, the ROM 6, and the chip selector 8 through an address bus 13 so that address data are supplied from the CPU 2 to the individual devices. Data terminals D$\phi$ to D7 of the CPU 2 are connected to the I/O port 4 and the ROM 6 through a data bus 15. Thus, data are read out from the ROM 6 and transmitted to the CPU 2, and data are transferred between the I/O port 4 and the CPU 2.

In a specific circuit arrangement, Z80 (by Zilog Co.) is used for the CPU 2; 8255A (Programmable I/O by Intel Corp.) for the I/O port 4; 2764 (by Intel Corp.) for the ROM 6; and SN74LS138 (by Texas Instrument Co.) for the chip selector 8. These devices are mounted on an IC substrate.

Input terminals PA$\phi$ and PA1 of the I/O port 4 are connected to nodes between pump output setting switches 14 and 16 and resistors 10 and 12, respectively. The resistors 10 and 12 are connected between a power source $V_{cc}$ and the ground. An input terminal PC1 of the I/O port 4 is connected with a zerocross detector 20 for detecting the zerocross point of AC voltage which is supplied from a commercial AC power source 18. The output terminal PB of the I/O port 4 from which a pump driving signal is delivered is connected to the base of a first transistor 24 through a resistor 22. The emitter of the transistor 24 is grounded, and its collector is connected to a power source $V_{DD}$ through the photodiode of a photocoupler 26 and a resistor 28. The commercial AC power source 18 is connected to a pump 30 through a Triac 32 for phase-controlling power supplied to the pump 30. A resistor 34 and the Triac of the photocoupler 26 are connected between the gate of the Triac 32 and a node between the Triac 32 and the pump 30 so that the Triac 32 is ignited at regular intervals.

The chip selector 8 is connected to the chip

selecting terminal $\overline{CS}$ of the I/O port 4 and the chip enabling terminal $\overline{CE}$ of the ROM 6, and the CPU 2 selects a chip in accordance with an instruction code. The memory readout signal terminal $\overline{RD}$ and machine cycle signal terminal $\overline{M1}$ of the CPU 2 are connected to an OR gate 36. The output of the OR gate 36 and the output of an inverter 37 connected to a chip enabling signal line are connected to a NOR gate 38, whose output is connected to a latch 40. The output of the latch 40 is connected to the base of a second transistor 44 through a resistor 42. The collector and emitter of the second transistor 44 are connected between the photodiode of the photocoupler 26 and the ground.

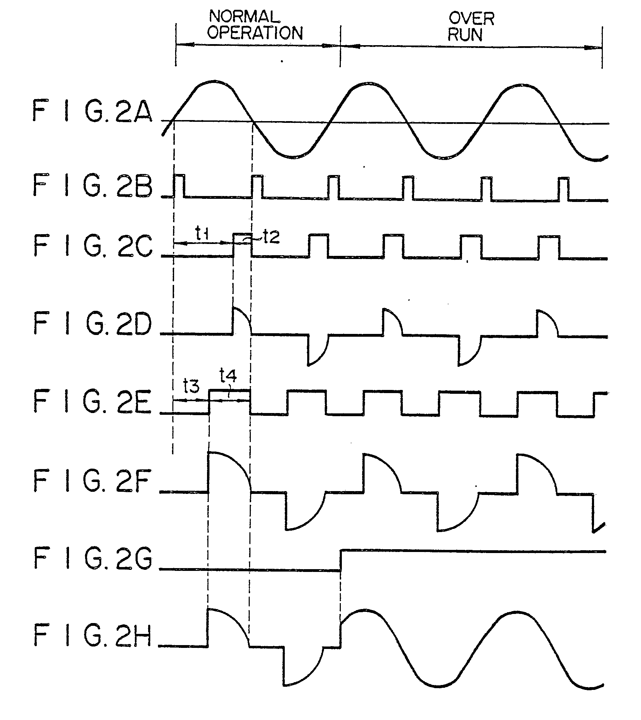

Constructed in this manner, the one embodiment of this invention operates as follows. When commercial AC supply voltage is supplied to the zerocross detector 20, as shown in Fig. 2A, the zerocross detector 20 detects the zerocross point, and produces a zerocross signal as shown in Fig. 2B. The produced zerocross signal is applied as an instruction code to the input terminal PC1 of the I/O port 4. Thereupon, when the pump output setting switch 16 is closed, a low level signal is supplied as an instruction code to the input terminal PAφ of the I/O port 4. The low level signal and the zerocross signal are fetched as instruction codes to the CPU 2 through the data bus 15. In response to a fetched instruction code for pump control, the CPU 2 causes the chip selector 8 to select the ROM 6 in which data for pump control is stored and the I/O port 4 for pump control. Thus, in response to an instruction code, prescribed data, i.e., data for a delay time t1, is read out from the ROM 6 with a prescribed address. The data read out in this manner is transferred from the ROM 6 to the selected I/O port 4. As a result, a low level signal is delivered from the output terminal PB of the I/O port 4 for the delay time t1 starting from a zerocross point, as shown in Fig. 2C, and then a high level signal is delivered for a time t2 directly following the time t1 and ending at the next zerocross point. Accordingly, the first transistor 24 is off for the delay time t1 and is on for the time t2. During the time t2 when the first transistor 24 is on, the photodiode of the photocoupler 26 emits light, and the photo-Triac of the photocoupler 26 is turned on to turn on the Triac 32. As a result, the pump 30 is supplied with power from the AC power source 18 for the time t2. Thus, a current as shown in Fig. 2D is continuously supplied to the pump 30 to secure a proper quantity of air supply, etc.

If the pump output setting switch 14 is closed instead of the switch 16, a delay time t3 is read out in place of the delay time t1, and an output signal as shown in Fig. 2E is delivered from the output terminal PB of the I/O port 4. Thus, a current as shown in Fig. 2F is supplied to the pump 30 to secure a modified proper quantity of air supply.

The CPU 2 can decide whether the frequency of the AC power source is 50 Hz or 60 Hz by counting zerocross signals as shown in Fig. 2B. A proper delay time is selected from data in the ROM 6 on the basis of the decision. Thus, whether the frequency is 50 Hz or 60 Hz, a specified quantity of air supply can be secured by the pump 30.

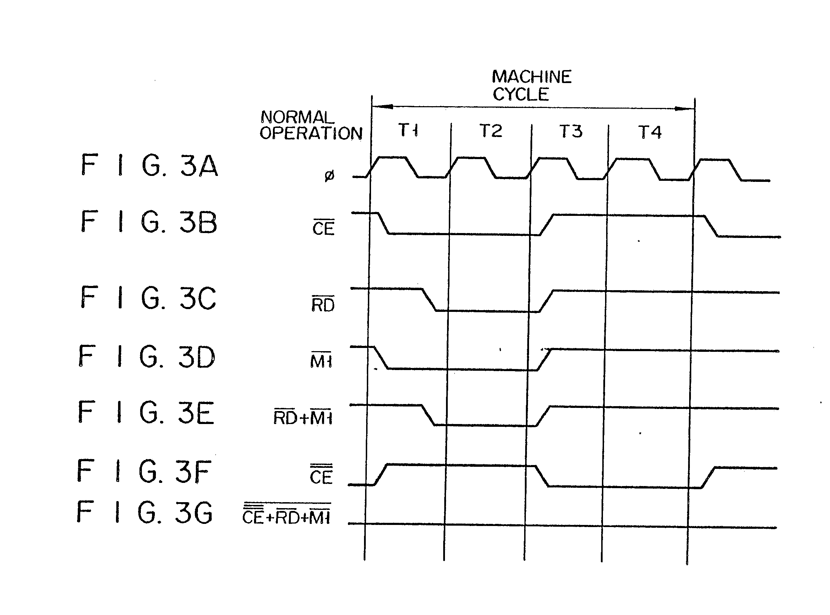

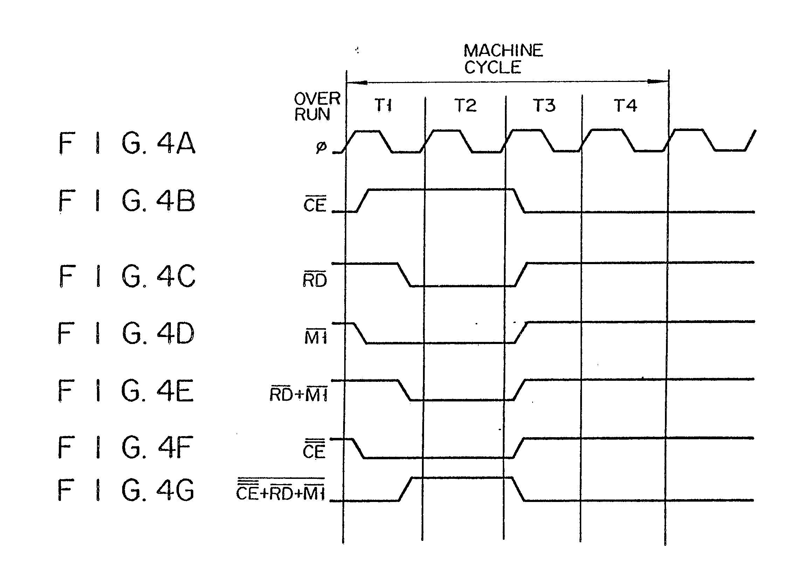

Referring now to Figs. 3A to 3G and 4A to 4G, there will be described timings of the CPU 2 for cases where the CPU2 operates normally and where it runs over due to noise or heat. Figs. 3A to 3G show timings in a machine cycle to fetch instruction codes. The CPU 2 is supplied with clock pulses as shown in Fig. 3A from the oscillator 10. A chip selecting signal is applied from the chip selector 8 to the chip selecting terminal $\overline{CS}$ of the I/O port 4. If the chip selecting signal goes low, the I/O port 4 is selected, and the I/O gate of the I/O port 4 is opened. Accordingly, an instruction code is fetched from the I/O port 4 to the CPU 2 by means of the data bus 13. A chip selecting signal as shown in Fig. 3B is applied from the chip selector 8 to the chip enabling terminal $\overline{CE}$ of the ROM 6. If this chip selecting signal goes low, the ROM 6 is selected and held ready for readout. Accordingly, an instruction code is fetched from the ROM 6 to the CPU 2 with a prescribed address. At this time, the memory readout terminal $\overline{RD}$ is maintained at low level, as shown in Fig. 3C. When the instruction code is fetched to the CPU 2, the machine cycle signal terminal $\overline{M1}$ is also maintained at low level, as shown in Fig. 3B. Therefore, while the output of the OR gate 36 is at low level, as shown in Fig. 3E, the CPU 2 reads instructions. Meanwhile, if the chip enabling terminal $\overline{CE}$ of the ROM 6 is at low level, data is read from the ROM 6. Thus, the output of the inverter 37 is as shown in Fig. 3F. As the outputs of the inverter 37 and the OR gate 36 are supplied to the NOR gate 38, the output of the NOR gate 38 is maintained at low level, as shown in Fig. 3G, and the second transistor 44 is kept off. In this state, current supply to the pump 30 is controlled in accordance with the on-off operation of the first transistor 24.

If the CPU 2 runs over, then the CPU 2 will fetch instruction data from some other memory region than the ROM 6. Namely, the chip selector 8 selects an empty region (e.g., a spare region for an additional ROM) on the IC mounting base on which some other chip than the ROM 6 is to be mounted, and instruction data is fetched from the empty region to the CPU 2. In other words, a high level non-enabling signal as shown in Fig. 4B is applied to the chip enabling terminal $\overline{CE}$ of the ROM 6, and instruction data is fetched to the CPU 2 also in a period during which the ROM 6 is not selected. Thus, the terminals $\overline{RD}$ and $\overline{M1}$ of the CPU 2 are maintained at low level, as shown in Figs. 4C and 4D. Accordingly, the outputs of the inverter 37 and the OR gate 36 are kept at low level, as shown in Figs. 4F and 4E, respectively, and a high level signal is delivered from the NOR gate 38, as shown in Fig. 4G. This high level signal is latched in the latch 40. As a result, a signal as shown in Fig. 2G is applied to the second transitor 44 to turn on the same. Thus, the Triac 32 is maintained in conduction, and the pump 30 is supplied with an AC current which is not phase-controlled, as shown in Fig. 2H, so that the maximum quantity of air supply is obtained. When an operator notices the over run of the microcomputer, he is to stop the operation of the system after suspending endoscopic diagnosis or treatment. At this time, if the operator depresses a reset button, a reset signal is applied to the latch 40 to clear the same.

In the aforementioned embodiment, an AC current is supplied directly to the pump 30 while the second transistor 44 is on. Alternatively, however, a phase-controlled AC current may be applied to the pump 30 so as to set a limited quantity of air supply.

According to the embodiment mentioned above, even if the CPU runs over, the pump can be securely driven to ensure stable air supply. Thus, the endoscope system can be improved in safety.

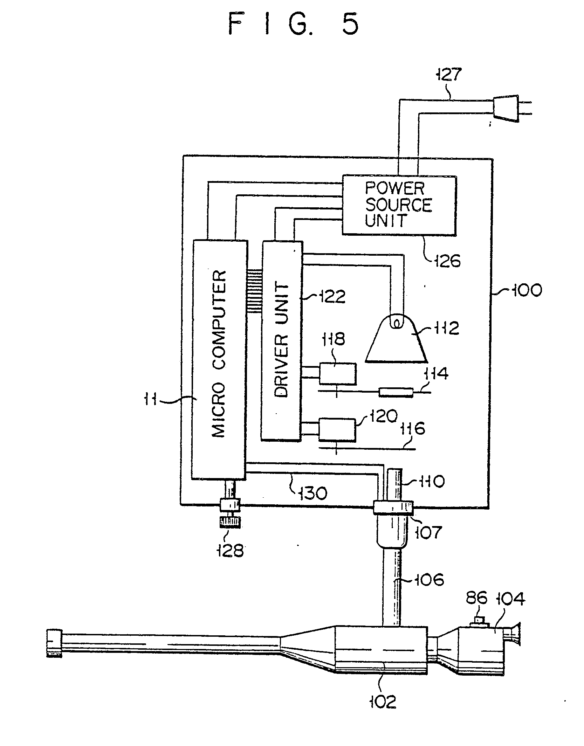

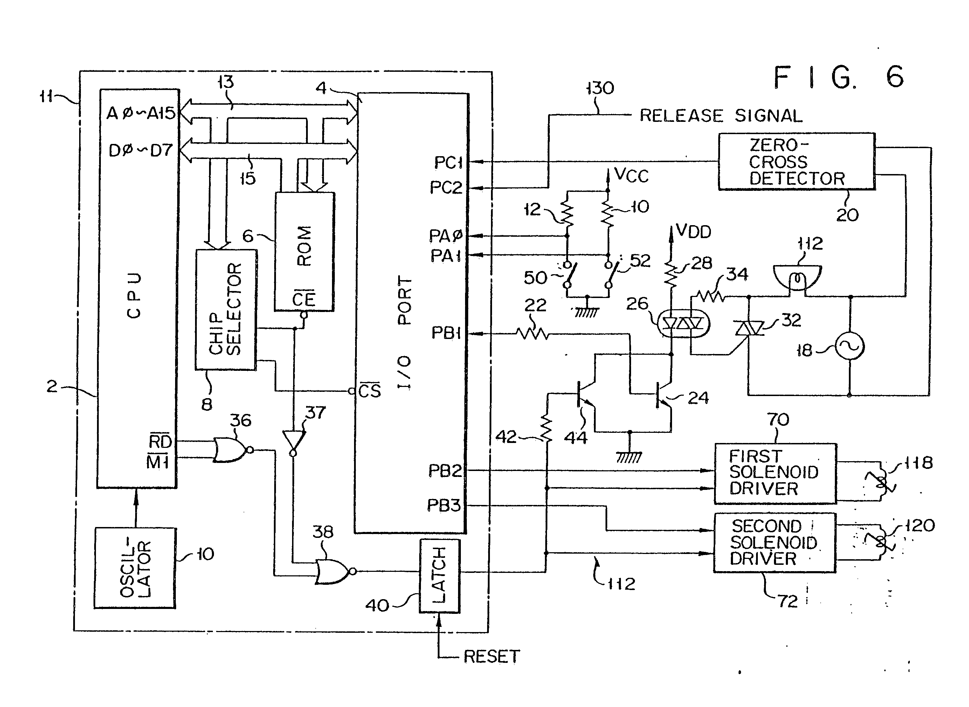

Referring now to Figs. 5 and 6, another embodiment of this invention will be described in detail. In Figs. 5 and 6, like reference numerals are used to designate the same portions as shown in Fig. 1. Fig. 5 schematically shows a light source unit 100 of an endoscope system. As is generally known, an endoscopic camera 104 is attached to the eyepiece section of an endoscope 102, and a universal cord 106 of the endoscope 102 is coupled to the light source unit 100 by means of a connector 107. Disposed in the light source unit 100 is a lamp 112 to supply illumination light and photographing light to the light incidence surface of a light guide 100 which extends through the universal cord 106. A color temperature changing filter 114 and a shutter 116 are arranged in a source light path extending from the lamp 112 to the incidence surface of the light guide 110. The filter 114 and the shutter 116 are coupled to solenoids 118 and 120 to drive them, respectively. The lamp 112 and the solenoids 118 and 120 are connected individually to a driver unit 122, which is connected to a CPU board or microcomputer 11. The driver unit 122 and the CPU board 11 are connected to a power source unit 126 from which extends a power supply cord 127 to be connected to a commercial power source. The CPU board 11 is also connected with a switch unit 128 for setting the brightness of the lamp 112, and is connected to a release signal line 130 which extends from the camera 104 through the universal cord 106.

Like the microcomputer 11 shown in Fig. 1, the CPU board 11 is composed of an ROM 6, a chip selector 8, an I/O port 4, etc. Thus, having the same construction as the one used in the embodiment of Fig 1, the CPU board 11 will not be described in detail below.

Input terminals PAɸ and PA1 of the I/O port 4 are connected to nodes between lamp brightness setting switches 50 and 52 of a switching unit 128 and resistors 10 and 12, respectively. The resistors 10 and 12 are connected between a power source $V_{cc}$ of a power source unit 126 and the ground. Input terminals PC2 and PC1 of the I/O port 4 are connected, respectively, with the release signal line 130 and a zerocross detector 20 for detecting the zerocross point of AC voltage which is supplied from a commercial AC power source 18 of the power source unit 126. An output terminal PB1 of the I/O port 4 from which a light source brightness setting signal is delivered is connected to the base of a first transistor 24 through a resistor 22. The emitter of the transistor 24 is grounded, and its collector is connected to a power source $V_{DD}$ through the photodiode of a photocoupler 26 and a resistor 28. The commercial AC power source 18 is connected to a lamp 112 through a Triac 32 for phase-controlling power supplied to the source lamp 112. A resistor 34 and the Triac of the photocoupler 26 are connected between the gate of the Triac 32 and a node between the Triac 32 and the source lamp 112 so that the Triac 32 is ignited at regular intervals.

Output terminals PB2 and PB3 of the I/O port 4 from which a filter insertion signal and a shutter switching signal are delivered, respectively, are connected to solenoid drivers 70 and 72, respectively. The solenoid drivers 70 and 72 are connected to solenoids 118 and 120 for filter and shutter, respectively. The output of a latch 40 is connected to the base of a second transistor 44 through a resistor 42, as well as to the solenoid drivers 70 and 72. The collector and emitter of the second transistor 44 are connected between the photodiode of the photocoupler 26 and the ground.

Constructed in this manner, the alternative embodiment of this invention shown in Figs. 5 and 6 operates as follows. When commercial AC supply voltage is supplied to the zero cross dector 20, as shown in Fig. 2A, the zero cross detector 20 detects the zero cross point, and produces a zero cross signal as shown in Fig. 2B. The produced zero cross signals is applied to the input terminal PC1 of the I/O port 4. Thereupon, when the lamp brightness setting switch 50 is closed, a low-level signal is supplied to the input terminal PAɸ of the I/O port 4. The low-level signal and the zero cross signal are fetched as instruction-codes to the CPU 2 through a data bus 15. In response to the fetched instruction codes, data for a programmed delay time t1 is read out from the ROM 6, and a low-level signal is delivered from the output terminal PB1 of the I/O port 4 for the delay time T1 starting from a zero cross point, as shown in Fig. 2C. Then a high level signal is delivered for a time T2 directly following the time t1 and ending at the next zerocross point. Accordingly, the first transistor 24 is off for the delay time t1 and on for the time t2. During the time t2 when the first transistor 24 is on, the photodiode of the photocoupler 26 emits light, and the photo-Triac of the photocoupler 26 is turned on to turn on the Triac 32. As a result, the lamp 112 is supplied with power from the AC power source 18 for the time t2. Thus, a current as shown in Fig. 2D is continuously supplied to the lamp 112 so that the lamp 112 glows with proper brightness. In this manner, sufficient illumination light can be supplied to the light guide 110 to secure satisfactory illuminated diagnosis. In the illuminated diagnosis, a filter removal signal and a shutter opening signal are applied from the CPU 2 to the output terminals PB2 and PB3, respectively, of the I/O port 4 to keep the solenoids 118 and 120 nonoperating, thereby removing the filter 114 and the shutter 116 from the light path.

If the light source brightness setting switch 52 is closed instead of the switch 50, a delay time t3 is read out from the ROM 6 in place of the delay time t1, and an output signal as shown in Fig. 2E is delivered from the output terminal PB1 of the I/O port 4. Thus, a current as shown in Fig. 2F is supplied to the source lamp 112 to secure modified proper light source brightness, e.g., higher brightness.

The CPU 2 can decide whether the frequency of the AC power source is 50 Hz or 60 Hz by counting zero cross signals as shown in Fig. 2B. A proper delay time is selected from data in the ROM 6 on the basis of the decision. Thus, whether the frequency is 50 Hz or 60 Hz, the source lamp 12 can be turned on to glow with specified brightness.

In photographing, when a release button 86 of the camera 104 is depressed, a synchro signal is applied to the input terminal PC2 of the I/O port 4 through the release signal line 130. When supplied with the synchro signal, the CPU 2 applies a filter insertion signal to the output terminal PB2 of the I/O port 4, and actuates the solenoid driver 70 to drive the solenoid 118, thereby locating the filter 114 in the light path. When the filter 114 is located in the light path, a mirror shutter (not shown) of the camera 104 is opened, and a film set in the camera 104 starts to be exposed. If the camera 104 includes a photometer, a shutter closing signal is applied from the CPU 2 to the output terminal PB3 of the I/O port 4 in response to an exposure stop signal delivered from the photometer. If the camera 104 is so designed that its exposure time is externally manually set, the shutter closing signal is applied after the passage of a programmed delay time. Accordingly, the solenoid driver 72 is actuated to drive the solenoid 120, thereby locating the shutter 116 in the source light path. Thus, film exposure is completed. When the mirror shutter of the camera 104 is closed, the filter 114 and the shutter 116 are removed from the light path to their original positions.

Referring now to Figs. 3A to 3G and 4A to 4G, there will be described timings of the CPU 2 for cases where the CPU 2 operates normally and where it runs over due to noise or heat. A signal as shown in Fig. 3B is applied from the chip selector 8 to the chip enabling terminal $\overline{CE}$ of the ROM 6. If this signal goes low, the ROM 6 is held ready for readout. When data is fetched from the ROM 6 to the CPU 2, as shown in Fig. 4C, the memory readout terminal $\overline{RD}$ is maintained at low level. When the instruction code is fetched to the CPU 2, the machine cycle signal terminal $\overline{M1}$ is also maintained at low level, as shown in Fig. 3D.

Therefore, while the output of the OR gate 36 is at low level, as shown in Fig. 3E, the CPU 2 reads instructions, Meanwhile, if the chip enabling terminal $\overline{CE}$ of the ROM 6 is at low level, data is read from the ROM 6. Thus, the output of the inverter 37 is as shown in Fig. 3F. As the outputs of the inverter 37 and the OR gate 36 are supplied to the NOR gate 38, the output of the NOR gate 38 is maintained at low level, as shown in Fig. 3G, and the second transistor 44 is kept off. Moreover, the solenoid drivers 70 and 72 are prevented from being returned to the nonoperating state by the output of the NOR gate 38. In this state, current supply to the lamp 112 is controlled in accordance with the on-off operation of the first transistor 24, and the solenoid drivers 70 and 72 are controlled by outputs from the output terminals PB2 and PB3 of the I/O port 4.

If the CPU 2 runs over, then the CPU 2 will fetch instruction data from some other memory region than the ROM 6. Namely, the chip enabling terminal $\overline{CE}$ of the ROM 6 is maintained at high level, and instruction data is fetched also in a period during which no chip is selected. Accordingly, the outputs of the inverter 37 and the OR gate 36 are kept at low level, and a high level signal is delivered from the NOR gate 38, as shown in Fig. 4G. This high level signal is maintained by the latch 40. As a result, a high level signal as shown in Fig. 4G is applied to the second transistor 44 and the solenoid drivers 70 and 72. Thus, the second transistor 44 is turned on to keep the Triac 26 in conduction and the solenoid drives 70 and 72 nonoperating, and the lamp 112 is supplied with an AC current which is not phase-controlled, as shown in Fig. 2H, so that the lamp is turned on to glow with maximum brightness. If the shutter 116 or the filter 114 is located in the source light path, therefore, it is removed from tha path to allow illumination light to be securely supplied to the region of interest.

It is to be understood that the light source 112 may glow not with the maximum brightness but with limited brightness.

According to the aforementioned embodiment, the illumination light continues to be supplied from the light source for the endoscope to the light guide even if the CPU runs over, so that a satisfactory visual field may be secured in the endoscope system.

Thus, according to this invention, there may be provided an endoscope system with high safety.

**Claims**

1. Safety circuit for a microprocessor controlled endoscope system comprising:
   a memory (6) in which control data is stored;
   a chip selector (8) for applying a chip enabling signal ($\overline{CE}$) to the memory (6) to hold the memory in an enabling state in which the control data can be read out from the memory (6);
   means (10, 12, 14, 16, 20) for producing setting signals;
   a microprocessor (2) for fetching the setting signals as instruction commands and reading out the control data in accordance with the setting signals, said processor (2) having a first terminal ($\overline{M1}$) from which a first logic signal is delivered while the instruction commands are being fetched, and a second terminal ($\overline{RD}$) from which a second logic signal is delivered while the control data is being read out from the memory (6);

a pump (30);

means (22, 24, 26, 28, 32) for adjusting power supplied to the pump (30) in accordance with the control data;

characterized by:

microprocessor failure detector means (36, 37, 38, 40) for producing an alarm logic signal while the chip selector (8) is not maintaining the memory (6) in the enabling state although the first and second logic signals are being produced; and

means (42, 44) for causing the adjusting means (22, 24, 26, 28, 32) to drive the pump (30) at a fixed power rate in response to the alarm signal.

2. Safety circuit according to claim 1, characterized in that said means (10, 12, 14, 16, 20) for producing the setting signals includes means (20) for discriminating power supply frequency and a switching circuit (10, 12, 14, 16).

3. Safety circuit according to claim 1, characterized in that said means (36, 37, 38, 40) for producing the alarm logic signal includes a logic circuit (36, 37, 38, 40) including a latch circuit (40) for latching the alarm logic signal.

4. Safety circuit according to claim 1, characterized in that said means (42, 44) for causing the pump adjusting means (22, 24, 26, 28, 32) to drive the pump (30) includes a switching element (44) to be closed in response to the alarm logic signal.

5. Safety circuit for a microprocessor controlled endoscope system comprising:

a memory (6) in which control data is stored;

a chip selector (8) for applying a chip enabling signal ($\overline{CE}$) to the memory (6) to hold the memory in an enabling state in which the control data can be read out from the memory (6);

means (10, 12, 14, 16, 20) for producing setting signals;

a microprocessor (2) for fetching the setting signals as instruction commands and reading out the control data in accordance with the setting signals, said processor (2) having a first terminal ($\overline{M1}$) from which a first logic signal is delivered while the instruction commands are being fetched, and a second terminal ($\overline{RD}$) from which a second logic signal is delivered while the control data is being read out from the memory (6);

a light source (112);

means (22, 24, 26, 28, 32) for adjusting power supplied to the light source (112) in accordance with the control data;

characterized by:

microprocessor failure detector means (36, 37, 38, 40) for producing an alarm logic signal while the chip selector (8) is not maintaining the memory (6) in the enabling state although the first and second logic signals are being produced; and

means (42, 44) for causing the adjusting means (22, 24, 26, 28, 32) to supply the light source (112) at a fixed power rate in response to the alarm signal.

6. Safety circuit according to claim 5, characterized in that said means (10, 12, 14, 16, 20) for producing the setting signals includes means (20) for discriminating power supply frequency and a switching circuit (10, 12, 14, 16).

7. Safety circuit according to claim 5, characterized in that said means (36, 37, 38, 40) for producing the alarm logic signal includes a logic circuit (36, 37, 38, 40) including a latch circuit (40) for latching the alarm logic signals.

8. Safety circuit according to claim 5, characterized in that said means (42, 44) for causing the adjusting means (22, 24, 26, 28, 32) to drive the light source (112) includes a switching element (44) to be closed in response to the alarm logic signal.

9. Safety circuit according to claim 5, characterized by further comprising a shutter (116) capable of being located in a source light path through which light from the light source (112) passes, and means (72, 120) for removing the shutter (116) from the source light path in response to an alarm signal.

10. Safety circuit according to claim 5, characterized by further comprising a filter (114) capable of being located in a source light path through which light from the light source (112) passes, and means (70, 118) for removing the filter (114) from the source light path in response to an alarm signal.

**Patentansprüche**

1. Sicherheitsschaltkreis für ein mikroprozessorgesteuertes Endoskopsystem, mit:

einem Speicher (6), in welchem Steuerdaten gespeichert sind;

einem Halbleiter-Anwähler (8) zur Zuführung eines Halbleiterfreigabesignales ($\overline{CE}$) zu dem Speicher (6), um den Speicher in einem aktivierten Zustand zu halten, in welchem die Steuerdaten aus dem Speicher (6) ausgelesen werden können;

Einrichtungen (10, 12, 14, 16, 20) zur Erzeugung von Setzsignalen;

einem Mikroprozessor (2) zum Empfang der Setzsignale als Anweisungsbefehle und zum Auslesen der Steuerdaten in Abhängigkeit der Setzsignale, wobei der Prozessor (2) einen ersten Anschluß ($\overline{M1}$) aufweist, von welchem ein erstes logisches Signal erhaltbar ist, wenn die Anweisungsbefehle aufgenommen werden und einen zweiten Anschluß ($\overline{RD}$) aufweist, von welchem ein zweites logisches Signal erhaltbar its, während die Steuerdaten aus dem Speicher (6) ausgelesen werden;

einer Pumpe (30);

Einrichtungen (22, 24, 26, 28, 32) um in Abhängigkeit der Steuerdaten die Energie, welche der Pumpe (30) zugeführt wird, einzustellen;

gekennzeichnet durch:

eine Erkennungsvorrichtung (36, 37, 38, 40) für Fehlverhalten des Mikroprozessors, um ein logisches Alarmsignal zu erzeugen, während der Halbleiter-Anwähler (8) den Speicher (6) nicht mehr im aktiven Zustand hält, obwohl die ersten und zweiten logischen Signale erzeugt werden; und

Einrichtungen (42, 44), welche die Einstellvorrichtungen (22, 24, 26, 28, 32) als Antwort auf das Alarmsignal veranlassen, die Pumpe (30) mit einer festgelegten Energiemenge zu betreiben.

2. Sicherheitsschaltkreis nach Anspruch 1, dadurch gekennzeichnet, daß die Einrichtungen (10, 12, 14, 16, 20) zur Erzeugung der Setzsignale eine Einrichtung (20) zur Diskriminierung der Energieversorgungsfrequenz und einen Schalterschaltkreis (10, 12, 14, 16) aufweisen.

3. Sicherheitsschaltkreis nach Anspruch 1, dadurch gekennzeichnet, daß die Einrichtungen (36, 37, 38, 40) zur Erzeugung des logischen Alarmsignales einen Logikschaltkreis (36, 37, 38, 40) aufweisen, der seinerseits einen Haltekreis (40) zum Zwischenspeichern des logischen Alarmsignales aufweist.

4. Sicherheitsschaltkreis nach Anspruch 1, dadurch gekennzeichnet, daß die Einrichtungen (42, 44), welche die Pumpeneinstellvorrichtungen (22, 24, 26, 28, 32) veranlassen, die Pumpe (30) anzutreiben, ein Schaltelement (44) aufweisen, welches als Antwort auf das logische Alarmsignal geschlossen wird.

5. Sicherheitsschaltkreis für ein mikroprozessorgesteuertes Endoskopsystem mit:

einer Speicher (6), in welchen Steuerdaten gespeichert sind;

einem Halbleiter-Anwähler (8) zur Zuführung eines Halbleiterfreigabesignales ($\overline{CE}$) zu dem Speicher (6), um den Speicher in einem aktivierten Zustand zu halten, in welchen die Steuerdaten aus dem Speicher (6) ausgelesen werden können;

Einrichtungen (10, 12, 14, 16, 20) zur Erzeugung von Setzsignalen;

einem Mikroprozessor (2) zum Empfang der Setzsignale als Anweisungsbefehle und zum Auslesen der Steuerdaten in Abhängigkeit der Setzsignale, wobei der Prozessor (2) einen ersten Anschluß ($\overline{M1}$) aufweist, von welchem ein erstes logisches Signal erhaltbar ist, wenn die Anweisungsbefehle aufgenommen werden und einen zweiten Anschluß ($\overline{RD}$) aufweist, von welchem ein zweites logisches Signal erhaltbar ist, während die Steuerdaten aus dem Speicher (6) ausgelesen werden;

einer Lichtquelle (112);

Einrichtungen (22, 24, 26, 28, 32) um in Abhängigkeit der Steuerdaten die Energie, welche der Lichtquelle (112) zugeführt wird, einzustellen;

gekennzeichnet durch:

eine Erkennungsvorrichtung (36, 37, 38, 40) für Fehlverhalten des Mikroprozessors, um ein logisches Alarmsignal zu erzeugen, während der Halbleiter-Anwähler (8) den Speicher (6) nicht mehr im aktiven Zustand hält, obwohl die ersten und zweiten logischen Signale erzeugt werden; und

Einrichtungen (42, 44), welche die Einstellvorrichtungen (22, 24, 26, 28, 32) als Antwort auf das Alarmsignal veranlassen, der Lichtquelle (112) eine festgelegte Energiemenge zuzuführen.

6. Sicherheitsschaltkreis nach Anspruch 5, dadurch gekennzeichnet, daß die Einrichtungen (10, 12, 14, 16, 20) zur Erzeugung der Setzsignale eine Einrichtung (20) zur Diskriminierung der Energieversorgungsfrequenz und einen Schalterschaltkreis (10, 12, 14, 16) aufweisen.

7. Sicherheitsschaltkreis nach Anspruch 5, dadurch gekennzeichnet, daß die Einrichtungen (36, 37, 38, 40) zur Erzeugung des logischen Alarmsignales einen Logikschaltkreis (36, 37, 38, 40) aufweisen, der seinerseits einen Haltekreis (40) zum Zwischenspeichern des logischen Alarmsignales aufweist.

8. Sicherheitsschaltkreis nach Anspruch 5, dadurch gekennzeichnet, daß die Einrichtungen (42, 44), welche die Einstellvorrichtungen (22, 24, 26, 28, 32) veranlassen, die Lichtquelle (112) anzutreiben, ein Schaltelement (44) aufweisen, welche als Antwort auf das logische Alarmsignal geschlossen wird.

9. Sicherheitsschaltkreis nach Anspruch 5, dadurch gekennzeichnet, daß weiterhin ein Verschluß (116) vorgesehen ist, der in einem Lichtquellenpfad anordenbar ist, entlang dem das Licht aus der Lichtquelle (112) läuft, und daß Einrichtungen (72, 120) vorgesehen sind, um den Verschluß (116) als Antwort auf das Alarmsignal aus dem Lichtquellenpfad zu entfernen.

10. Sicherheitsschaltkreis nach Anspruch 5, dadurch gekennzeichnet, daß weiterhin ein Filter (114) vorgesehen ist, welches in dem Lichtquellenpfad anordenbar ist, entlang dem das Licht aus der Lichtquelle (112) läuft, und daß Einrichtungen (70, 118) vorgesehen sind, um das Filter (114) als Antwort auf das Alarmsignal aus dem Lichtquellenpfad zu entfernen.

**Revendications**

1. Circuit de sécurité pour système endoscopique commandé par microprocesseur comprenant:

une mémoire (6) dans laquelle les données de contrôle sont stockées;

un sélecteur semi-conducteur (8) pour amener un signal de libération semi-conducteur ($\overline{CE}$) à la mémoire (6) pour maintenir la mémoire dans un état activé dans lequel les données de commande peuvent être lues de la mémoire (6);

des dispositifs (10, 12, 14, 16, 20) pour produire des signaux d'activation;

un microprocesseur (2) pour recevoir les signaux d'activation comms instructions et lire les données de commande en fonction des signaux d'activation, ledit processeur (2) ayant un premier raccord ($\overline{M1}$) fournissant un premier signal logique lorsque les instructions sont reçues, et un deuxième raccord ($\overline{RD}$) fournissant un deuxième signal logique lorsque les données de contrôle sont lues de la mémoire (6);

une pompe (30);

des dispositifs (22, 24, 26, 28, 32) pour ajuster l'énergie fournie à la pompe (30) en fonction des données de contrôle; caractérisé par:

un dispositif détecteur de défaillances du microprocesseur (36, 37, 38, 40) pour produire un signal d'alarme logique lorsque le sélecteur semi-conducteur (8) ne maintient plus la mémoire (6) dans un état activé, bien que le premier et le deuxième signal logique aient été produits; et

des dispositifs (42, 44) qui font en sorte que les dispositifs d'ajustage (22, 24, 26, 28, 32) activent la pompe (30) avec une quantité d'énergie fixe, en réponse au signal d'alarme.

2. Circuit de sécurité selon la revendication 1, caractérisé en ce que lesdits dispositifs (10, 12, 14, 16, 20) pour produire les signaux d'activation incluent un dispositif (20) pour discriminer la fréquence d'approvisionnement en énergie et un circuit de commutation (10, 12, 14, 16).

3. Circuit de sécurité selon la revendication 1, caractérisé en ce que lesdits dispositifs (36, 37, 38, 40) pour produire le signal d'alarme logique incluent un circuit logique (36, 37, 38, 40) comprenant un circuit d'arrêt (40) pour enregistrer le signal d'alarme logique.

4. Circuit de sécurité selon la revendication 1, caractérisé en ce que les dispositifs (42, 44) qui font en sorte que les dispositifs d'ajustage de la pompe (22, 24, 26, 28, 32) activent la pompe (30) incluent un élément de commutation (44) qui est fermé en réponse au signal d'alarme logique.

5. Circuit de sécurité pour système endoscopique commandé par microprocesseur comprenant:

une mémoire (6) dans laquelle les données de contrôle sont stockées;

un sélecteur semi-conducteur (8) pour amener un signal de libération semi-conducteur ($\overline{CE}$) à la mémoire (6) pour maintenir la mémoire dans un état activé dans lequel les données de commande peuvent être lues de la mémoire (6);

des dispositifs (10, 12, 14, 16, 20) pour produire des signaux d'activation;

un microprocesseur (2) pour recevoir les signaux d'activation comme instructions et lire les données de commande en fonction des signaux d'activation, ledit processeur (2) ayant un premier raccord ($\overline{M1}$) fournissant un premier signal logique lorsque les instructions sont reçues, et un deuxième raccord ($\overline{RD}$) fournissant un deuxième signal logique lorsque les données de contrôle sont lues de la mémoire (6);

une source lumineuse (112);

des dispositifs (22, 24, 26, 28, 32) pour ajuster l'énergie fournie à la source lumineuse (112) en fonction des données de contrôle; caractérisé par:

un dispositif détecteur de défaillances du microprocesseur (36, 37, 38, 40) pour produire un signal d'alarme logique lorsque le sélecteur semi-conducteur (8) ne maintient plus la mémoire (6) dans un état activé, bien que le premier et le deuxième signal logique aient été produits; et

des dispositifs (42, 44) qui font en sorte que les dispositifs d'ajustage (22, 24, 26, 28, 32) amènent à la source lumineuse (112) une quantité d'énergie fixe, en réponse au signal d'alarme;

6. Circuit de sécurité selon la revendication 5, caractérisé en ce que lesdits dispositifs (10, 12, 14, 16, 20) pour produire les signaux d'activation incluent un dispositif (20) pour discriminer la fréquence d'approvisionnement en énergie et un circuit de commutation (10, 12, 14, 16).

7. Circuit de sécurité selon la revendication 5, caractérisé en ce que lesdits dispositifs (36, 37, 38, 40) pour produire le signal d'alarme logique incluent un circuit logique (36, 37, 38, 40) comprenant un circuit d'arrêt (40) pour enregistrer le signal d'alarme logique.

8. Circuit de sécurité selon la revendication 5, caractérisé en ce que les dispositifs (42, 44) qui font en sorte que les dispositifs d'ajustage (22, 24, 26, 28, 32) activent la source lumineuse (112) incluent un élément de commutation (44) qui est fermé en réponse au signal d'alarme logique.

9. Circuit de sécurité selon la revendication 5, caractérisé en ce qu'il comprend en outre une fermeture (116) pouvant être placée dans une voie de source lumineuse le long de laquelle la lumière de la source lumineuse (112) passe, et des dispositifs (72, 120) pour enlever la fermeture (116) de la voie de source lumineuse en réponse au signal d'alarme.

10. Circuit de sécurité selon la revendication 5, caractérisé en ce qu'il comprend en outre un filtre (114) pouvant être placé dans une voie de source lumineuse le long de laquelle la lumière de la source lumineuse (112) passe, et des dispositifs (70, 118) pour enlever le filtre (114) de la voie de source lumineuse en réponse au signal d'alarme.

F I G. 1

0 078 035

FIG.2A
FIG.2B
FIG.2C
FIG.2D
FIG.2E
FIG.2F
FIG.2G
FIG.2H

NORMAL OPERATION

OVER RUN

2

F I G. 3A

F I G. 3B

F I G. 3C

F I G. 3D

F I G. 3E

F I G. 3F

F I G. 3G

NORMAL OPERATION

MACHINE CYCLE

| T1 | T2 | T3 | T4 |

$\emptyset$

$\overline{CE}$

$\overline{RD}$

$\overline{M1}$

$\overline{RD} + \overline{M1}$

$\overline{\overline{CE}}$

$\overline{\overline{\overline{CE} + \overline{RD} + \overline{M1}}}$

0 078 035

MACHINE CYCLE

OVER RUN | T1 | T2 | T3 | T4

F I G. 4A    ø

F I G. 4B    $\overline{CE}$

F I G. 4C    $\overline{RD}$

F I G. 4D    $\overline{M1}$

F I G. 4E    $\overline{RD}+\overline{M1}$

F I G. 4F    $\overline{\overline{CE}}$

F I G. 4G    $\overline{\overline{\overline{CE}+\overline{RD}+\overline{M1}}}$

0 078 035

# F I G. 5

F I G. 6

0 078 035